# FASCICULE DE BREVET EUROPEEN

(11) **EP 3 498 627 B1**
(45) Date de publication et mention de la délivrance du brevet: **04.03.2020**
(21) Numéro de dépôt: 18205224.1
(22) Date de dépôt: 08.11.2018
(51) Int. Cl.: B65D 65/10, B65B 11/54, B65B 23/00, B65D 75/00, B65D 75/14, B65B 11/00, B65D 65/00, G03B 42/04, B65B 33/02, A61B 6/00

(54) **PROCEDE D'EMBALLAGE D'UN BOITIER ET CASSETTE RADIOLOGIQUE NUMERIQUE PORTABLE COMPRENANT UN BOITIER**
VERFAHREN ZUM VERPACKEN EINES GEHÄUSES UND TRAGBARE DIGITALE RÖNTGENKASSETTE MIT EINEM GEHÄUSE
METHOD FOR PACKAGING A HOUSING AND PORTABLE DIGITAL X-RAY CASSETTE COMPRISING A HOUSING

(30) Priorité: 14.12.2017 FR 1762131
(43) Date de publication de la demande: 19.06.2019
(73) Titulaire: Trixell, 38430 Moirans (FR)
(72) Inventeur: LE BECHENNEC, Philippe, 38430 MOIRANS (FR); TUAILLON, Cédric, 38430 MOIRANS (FR)
(74) Mandataire: Derval, Estelle

(56) Documents cités:
- FR-A1- 3 000 344
- US-A1- 2010 047 529
- US-A1- 2016 122 120

## Description

L'invention concerne une cassette radiologique portable destinée à équiper un système radiologique numérique. La cassette comprend un détecteur numérique de rayonnement ionisant permettant de fournir une image fonction du rayonnement reçu.

Le système radiologique comprend en outre une source de rayonnement ionisant, comme par exemple un tube à rayons X, permettant de générer un rayonnement X et une station de base comprenant un système de traitement de l'information permettant de synchroniser le tube à rayon X et le détecteur et permettant aussi de réaliser des traitements d'images comme de présenter à l'opérateur l'image corrigée de tous les défauts inhérents au détecteur et améliorée, par exemple par des traitements de rehaussement de contour. Un objet dont on veut obtenir l'image X est placé entre la source et le détecteur. Un tel système peut être utilisé dans de nombreuses applications telles que par exemple la radiologie médicale et le contrôle non destructif. L'invention peut également être mise en œuvre pour d'autres types de rayonnements à détecter notamment des rayonnements gamma.

Par le passé, les systèmes radiologiques étaient volumineux et peu mobiles. Il était nécessaire de positionner l'objet par rapport au système pour obtenir l'image désirée. Avec l'apparition de détecteurs à l'état solide tels que par exemple décrit dans la demande de brevet français FR 2 605 166, le détecteur est devenu moins volumineux et il a été possible de déplacer le détecteur par rapport à un objet restant fixe. Pour la radiologie médicale on a réalisé des détecteurs numériques sous forme de cassettes mobiles qu'il devient possible de placer à proximité immédiate d'un patient dont on veut réaliser une image, lorsque l'état de santé du patient empêche son déplacement vers une salle réservée à la radiologie.

La cassette portable comprend un boîtier assurant principalement la fonction de support mécanique et de protection du détecteur des déformations lors des chutes. Le boîtier possède une forme essentiellement parallélépipédique. Le rayonnement à détecter traverse le boîtier par une de ses plus grandes faces.

La surface extérieure du boîtier peut être laissée brute ou bien être peinte. Le fait de laisser la surface extérieure brute apporte souvent une esthétique peu satisfaisante, et une mauvaise aptitude au nettoyage qui doit être fréquent en cas d'utilisation de la cassette en radiologie médicale. Le fait de peindre la surface extérieure du boîtier permet d'améliorer l'esthétique mais la robustesse reste limitée face à des agressions mécaniques telles que des chocs, des rayures ou encore l'abrasion due aux manipulations répétées de la cassette.

Une solution alternative consiste à recouvrir le boîtier d'un revêtement à base de films plastiques assurant l'étanchéité aux liquides et aux poussières de la cassette.

Ce type de revêtement améliore aussi la résistance mécanique de la surface extérieure de la cassette face aux rayures et à l'abrasion et permet de résister aux agressions chimiques, notamment des produits de nettoyage et de désinfection. Pour la face exposée au rayonnement, il présente une atténuation minimale et homogène du rayonnement.

Par ailleurs, la cassette, lors de son utilisation en radiologie médicale, est amenée à être déposé sur des dessertes et plus généralement sur du mobilier hospitalier. La cassette doit présenter un coefficient de frottement suffisant pour s'arrêter rapidement si elle est déposée avec une certaine vitesse et rester en place une fois déposée, même sous des sollicitations accidentelles. Ceci implique un coefficient de frottement suffisant. Inversement, dans les phases d'utilisation, le coefficient de frottement doit être assez bas pour permettre de glisser le détecteur sous un patient sans effort exagéré. Un film est beaucoup plus résistant aux agressions mécaniques qu'une peinture, présente un minimum d'aspérités et de zones de rétention pénalisant son aptitude au nettoyage, et peut permettre à terme une rénovation de l'aspect extérieur de la cassette par remplacement des films. Le remplacement est notamment utile lors de la présence de rayures dans lesquelles des impuretés peuvent s'accumuler.

Une solution divulguée dans la demande de brevet française portant le numéro de publication FR3000344, consiste à recouvrir successivement chacune des plus grandes faces du boîtier d'un film thermoplastique et de recouvrir trois faces latérales du boîtier et de faire déborder ces films sur trois faces latérales du boîtier en leur faisant épouser la surface du boîtier par thermoformage à une température de l'ordre de 110°C. Le thermoformage est réalisé sous vide de sorte à assurer le placage du film sur le boîtier et éviter la formation de plis. Les deux films se recouvrent sur les faces latérales du boîtier.

Ce procédé présente un certain nombre d'inconvénients.

Lors de la phase de thermoformage, chaque film est étiré et est donc aminci, en particulier dans les coins du boîtier, là où le boîtier est soumis à des sollicitations mécaniques fortes qui le fragilisent ce qui provoque son vieillissement prématuré. L'étirement des films provoque également un amincissement de l'épaisseur de la colle sur le pourtour des plus grandes faces ce qui limite la robustesse de la protection apportée par les films. La sollicitation des films en température a tendance à les rendre cassants ce qui entraine leur vieillissement prématuré. Par ailleurs, le boîtier présente classiquement des parties en aluminium sur lesquelles les films collent difficilement. En effet, l'aluminium, qui est un bon conducteur thermique, fait obstacle au durcissement de la colle qui reste localement à une température trop faible.

Le thermoformage nécessitant un chauffage à haute température, la personnalisation de la cassette par impression ne peut être faite qu'après la phase de collage sur le film ce qui fragilise cette personnalisation qui est soumise à sollicitations importantes. Par ailleurs, ce procédé est coûteux et long, il nécessite des équipements lourds notamment pour faire le vide.

Un but de l'invention est de limiter au moins un des inconvénients précités.

A cet effet, l'invention a pour objet un procédé d'emballage d'un boîtier présentant une forme essentiellement parallélépipédique définie par des faces principales dont une première plus grande face et une deuxième plus grande face et des faces latérales, les faces latérales étant séparées deux à deux par des coins du boîtier, le boîtier logeant un détecteur numérique de rayonnement ionisant sous forme d'un panneau plat, le procédé comprenant une étape principale de recouvrement du boîtier par plusieurs films comprenant une première étape de recouvrement de la première plus grande face du boîtier par un premier film configuré, positionné et orienté par rapport à la première plus grande face de sorte à comprendre des rabats débordant de la première plus grande face et aptes à être repliés indépendamment l'un de l'autre pour venir recouvrir des faces latérales adjacentes.

Avantageusement, le premier film comprend au moins trois rabats débordant de la première grande face et aptes à être repliés indépendamment les uns des autres pour venir recouvrir au moins trois des faces latérales du boîtier.

Avantageusement, le procédé comprend une étape de pliage des rabats de sorte que les rabats viennent recouvrir des faces latérales adjacentes du boîtier.

Avantageusement, les rabats débordent des faces latérales adjacentes, le procédé comprenant une étape de pliage des rabats de sorte qu'un reste des rabats vienne recouvrir la deuxième plus grande face.

Avantageusement, l'étape principale de recouvrement comprend une étape recouvrement de la deuxième plus grande face par un deuxième film, le deuxième film recouvrant uniquement la deuxième plus grande face.

Avantageusement, l'étape principale de recouvrement est réalisée de sorte que les deux films ne se recouvrent pas, le procédé comprenant une étape de colmatage d'un interstice entre le premier film et le deuxième film par un joint.

Avantageusement, l'étape principale de recouvrement comprend une étape de découpage et de retrait de bords des restes des rabats recouvrant la deuxième plus grande face selon une ligne de découpe de position prédéterminée par rapport à la deuxième plus grande face et une étape de découpe et de retrait d'un bord du deuxième film selon une deuxième ligne de découpe fermée de position et de forme prédéterminée par rapport à la première ligne de découpe.

Avantageusement, le procédé comprend une étape de recouvrement des coins du boîtier par des protections de coins.

Avantageusement, le procédé comprend une étape de découpage et de retrait de bords du premier film entourant des coins du boîtier de façon à laisser libres des parties du boîtier comprenant et entourant des coins du boîtier, les protections de coins étant disposées de façon à recouvrir les parties libres sans recouvrir le premier film.

Avantageusement, les épaisseurs de chaque protection de coin et de chaque film sont choisies de sorte que lorsque la cassette est posée sur un support plan, seules les protections de coins sont en contact avec le support plan.

Avantageusement, au moins un film comprend un film plastique, une couche d'assemblage permettant de faire adhérer le premier film au boîtier et une couche d'impression interposée entre un film plastique et la couche d'assemblage.

L'invention se rapporte également à une cassette radiologique portable comprenant un boîtier présentant une forme essentiellement parallélépipédique définie par des faces principales dont une première plus grande face et une deuxième plus grande face et des faces latérales, les faces latérales étant séparées deux à deux par des coins du boîtier, le boîtier logeant un détecteur numérique de rayonnement ionisant sous forme d'un panneau plat, le boîtier étant recouvert par plusieurs films comprenant un premier film recouvrant la première grande face, le premier film comprenant des rabats débordant de la première plus grande face et repliés indépendamment l'un de l'autre pour recouvrir des faces latérales adjacentes.

Avantageusement, un reste de chaque rabat recouvre la deuxième plus grande face.

Avantageusement, la cassette comprend un deuxième film recouvrant uniquement la deuxième plus grande face.

Avantageusement, la cassette radiologique comprend un joint colmatant un interstice entre le premier film et le deuxième film.

Avantageusement, la cassette radiologique comprend des protections de coins recouvrant les coins.

Avantageusement, les protections de coins ne recouvrent pas les films.

Avantageusement, l'épaisseur de chaque protection de coin et de chaque film est choisie de sorte que lorsque la cassette est posée sur un support plan, seules les protections de coins sont en contact avec le support plan.

Avantageusement, au moins un film comprend un film plastique, une couche d'assemblage permettant de faire adhérer le premier film au boîtier et une couche d'impression interposée entre un film plastique et la couche d'assemblage.

L'invention sera mieux comprise et d'autres avantages apparaîtront à la lecture de la description détaillée d'un mode de réalisation donné à titre d'exemple, description illustrée par le dessin joint dans lequel :
la figure 1 représente un système radiologique mettant en œuvre l'invention ;
la figure 2 représente en perspective un boîtier d'une cassette selon l'invention
les figures 3a à 3k représentent un boitier puis les produits intermédiaires obtenus après les différentes étapes du procédé selon l'invention ainsi que la cassette en vue de dessus face à une première plus grande face du parallélépipède défini par le boitier (figures 3a, 3b) et face à la deuxième plus grande face du parallélépipède défini par le boitier (figures 3c à 3k) ;
la figure 4 représente schématiquement une vue en coupe du produit intermédiaire de la figure 3i selon le plan de coupe AA,
la figure 5 représente schématiquement une vue en coupe d'un première film F1
la figure 6 représente schématiquement une vue en coupe de la cassette selon le plan de coupe BB de la figure 3k.

Par souci de clarté, les mêmes éléments porteront les mêmes repères dans les différentes figures.

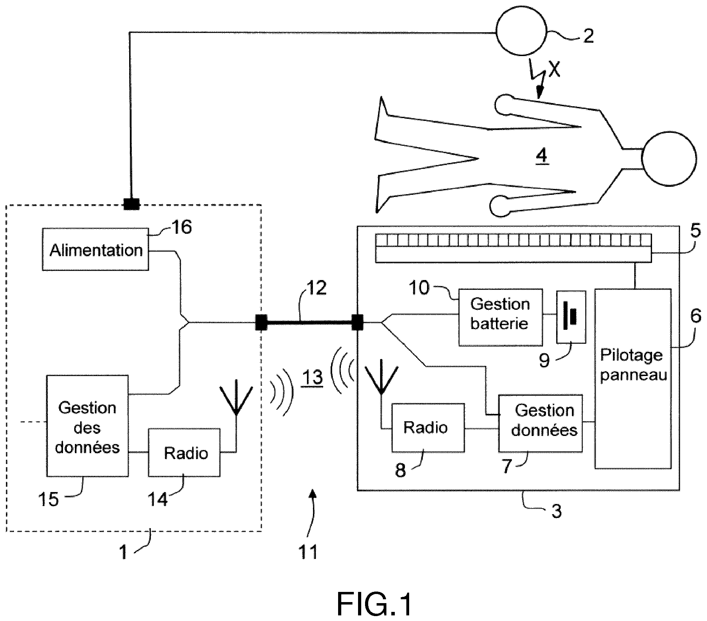

La figure 1 représente un système radiologique destiné à une utilisation médicale. Le système comporte une station de base fixe 1, un générateur de rayonnement X 2 et un détecteur de rayonnement sous forme d'une cassette portable 3. La cassette permet d'obtenir une image d'un patient 4 traversé par le rayonnement X issu du générateur 2. La cassette 3 comporte un détecteur numérique réalisé sous forme d'un panneau plat 5 relié à un module de pilotage 6 permettant de lire l'image obtenue par le panneau plat 5 et de la numériser au travers d'un convertisseur analogique numérique. La cassette mobile 3 comporte également un module de gestion de données 7, un module radio 8, une batterie 9 et un module de gestion de la batterie 10.

La station de base comporte un module radio 14, un module de gestion de données 15 et une alimentation 16.

Des moyens de communication 11 entre la cassette 3 et la station de base 1 permettent de transférer des données telles que l'image entre la cassette 3 et la station de base 1. Les données peuvent circuler soit de la station de base 1 vers la cassette 3, soit de la cassette 3 vers la station de base 1. Vers la cassette 3, il s'agit par exemple d'informations de commande du panneau plat 5 et vers la station de base 1, les données comportent par exemple des images réalisées par le panneau plat 5.

Les moyens de communication peuvent comprendre une liaison filaire amovible 12 et/ou une liaison sans fil 13. Les deux liaisons 12 et 13 sont susceptibles toutes deux de transférer les données. Les deux modules radio 8 et 14 permettent d'échanger les données entre la station de base 1 et la cassette 3. Le module de gestion de données 7 de la cassette 3 permet d'aiguiller les données reçues ou en provenance du module de pilotage 6 vers l'une des liaisons 12 ou 13. De même, dans la station de base 1, le module de gestion de données 15 permet d'aiguiller les données reçues ou en provenance d'une des liaisons 12 ou 13. L'alimentation 16 fournit l'énergie électrique nécessaire au fonctionnement des différents modules de la station de base 1 ainsi que de la cassette 3.

L'alimentation de la cassette 3 se fait par l'intermédiaire de la liaison filaire 12 ou de la batterie 9. Avantageusement, le système comporte des moyens pour recharger la batterie 9. Plus précisément, le module de gestion de la batterie 10 mesure la charge de la batterie 9 et provoque sa recharge en cas de besoin.

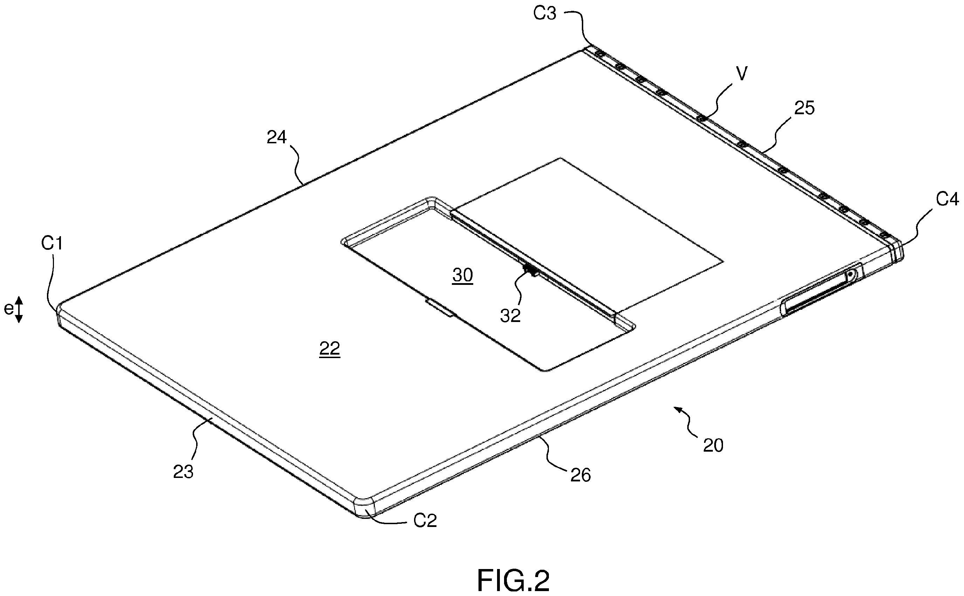

La figure 2 représente un boîtier 20 de la cassette radiologique 3 selon l'invention. Le boîtier 20 a une forme essentiellement parallélépipédique. Le détecteur numérique 5, le module de pilotage 6, le module de gestion de données 7, le module radio 8, et le module de gestion de la batterie 10 sont disposés à l'intérieur du boîtier 20. Ces quatre modules sont disposés sur une carte électronique. Les modules ne sont donnés qu'à titre d'exemple. Ils ne sont pas obligatoires pour la mise en œuvre de l'invention. La batterie 9 est disposée à l'extérieur du boîtier 20 afin de faciliter un éventuel remplacement

Le boîtier 20 possède six faces principales 21 à 26 délimitant la forme sensiblement parallélépipédique. Les six faces sont parallèles deux à deux. Les plus grandes faces principales 21, 22 sont reliées entre elles par les coins du boîtier C1, C2, C3, C4 séparés deux à deux par les faces latérales 23 à 26. Le détecteur 5, sous forme d'un panneau plat, possède une surface de détection de rayonnement proche de celle des deux plus grandes faces 21 et 22 parallèles l'une à l'autre. Les autres faces 23 à 26 sont les plus petites faces du boîtier 20. Sur la réalisation des figures, le parallélépipède est essentiellement rectangulaire. En variante, les faces du parallélépipède ne sont pas rectangulaires.

La batterie 9 est logée à l'intérieur du volume parallélépipédique formée par le boîtier 20. La batterie 9 est logée par l'extérieur du boîtier 20 dans un embrèvement 30 réalisé dans la face 22. L'embrèvement 30 forme un creux dans la deuxième plus grande face 22, creux complémentaire de la forme de la batterie 9 de façon qu'une fois mise en place, la batterie 9 ne dépasse pas de la face 22.

Le détecteur numérique 5 est disposé à l'intérieur du boîtier 20 du coté de la face 21. La face 21 est sensiblement plane.

La cassette 3 comprend au moins un connecteur 32 disposé en traversée d'une paroi du boîtier 20.

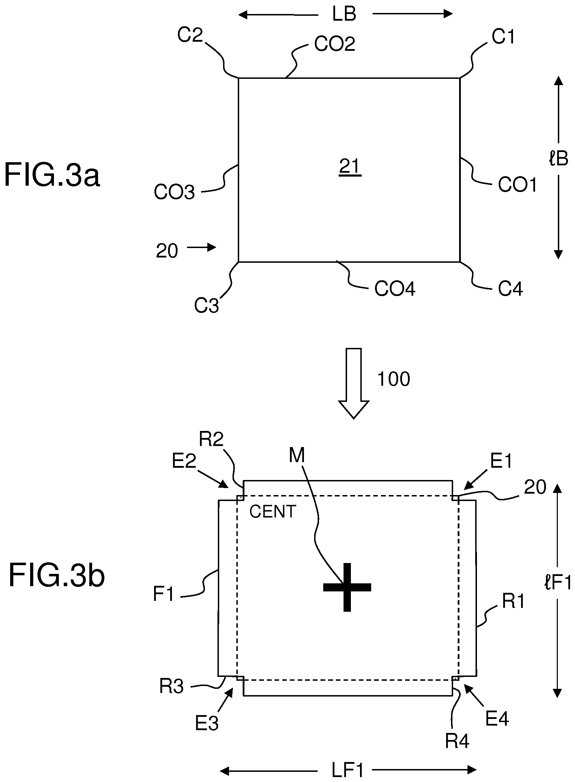

Les figures 3a à 3k représentent le produit initial (figures 3a) puis les produits intermédiaires obtenues lors des différentes étapes (représentées par des flèches) du procédé selon l'invention de façon à obtenir la cassette radiologique 3 selon l'invention.

Le procédé d'emballage consiste à venir recouvrir le boîtier d'un revêtement plaqué sur le boîtier.

Sur ces schémas, pour plus de clarté, le boîtier 20 est représenté par un parallélépipède rectangle parfait dépourvu d'embrèvement.

Le procédé d'emballage selon l'invention comprend une étape principale de recouvrement pour recouvrir le boitier de plusieurs films.

Cette étape principale de recouvrement comprend une première étape 100 de recouvrement de la première plus grande face 21 par un premier film F1. Dans le procédé selon l'invention, lors d'une étape de recouvrement d'une face principale du parallélépipède par un film, on fait adhérer le film à la face principale et le film est plaqué sur la face principale sur toute la partie de sa surface recouvrant la face principale.

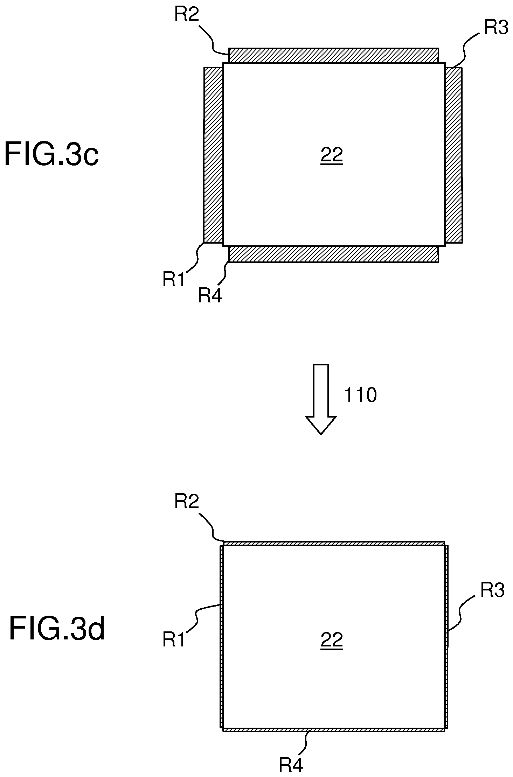

Le premier film F1 est configuré mais aussi positionné et orienté par rapport à la première grande face 21 de sorte à comprendre des rabats R1, R2, R3, R4 débordant de la première grande face 21 au-delà des quatre côtés CO1, CO2, CO3, CO4 de la première grande face 21 comme cela est représenté sur les figures 3b et 3c représentant le boîtier 20 recouvert du premier film F1 à l'issu de l'étape 100 de face, face à la première grande face 21 (figure 3b) et face à la deuxième face 22 (figure 3c). Par configuration du film, on entend notamment ses dimensions et forme.

Comme visible sur les figures 3b et 3c, chaque rabat R1, R2, R3 et R4 déborde de la première grande face 21 et est apte à être replié indépendamment des autres rabats pour venir recouvrir une des faces latérales 23 à 26 du boîtier 20.

Autrement dit, une fois que le premier film F1 recouvre la première face 21, il est fixé et accolé à cette première face 21 dans la position définie ci-dessus et représentée sur la figure 1, il est possible de venir replier chaque rabat R1, R2, R3 et respectivement R4 le long d'un des côtés CO1, CO2, CO3, et respectivement CO4 de la grande face 21 sans replier les autres rabats. Ainsi, les rabats adjacents sont séparés l'un de l'autre de façon que chacun de ces rabats puisse être replié selon un des côtés CO1, CO2, CO3, ou respectivement CO4 du boîtier séparant la première plus grande face 21 d'une des faces latérales 23, 24, 25 ou 26. Les deux rabats adjacents sont repliés selon des bords présentant des directions différentes de façon à venir recouvrir des faces latérales adjacentes tout en étant plaqués sur le boîtier sur toutes leurs surfaces. Ce procédé permet de venir recouvrir les différentes faces latérales 23 à 26 du boîtier 20 sans thermoformage et donc d'éviter les inconvénients associés au thermoformage. Il est possible de venir recouvrir chaque face latérale 23 à 26 du boîtier uniquement par pliage tout en assurant un placage continu du film sur le boîtier.

Ainsi le procédé d'emballage selon l'invention permet de garantir une épaisseur constante de film sur toute sa surface, de garantir une tenue mécanique sur l'aluminium bien supérieure à celle d'un film thermoformé, de garantir une épaisseur constante d'adhésif sur toute sa surface collée au boîtier, de ne pas faire subir de vieillissements prématurés au film. Il permet donc d'augmenter la robustesse du détecteur vis-à-vis de l'environnement, de faciliter et améliorer le nettoyage. Il permet également de diminuer les coûts et temps de fabrication de la cassette. La mise sous vide n'est pas nécessaire pour venir plaquer le film, sur toute sa surface, sur le boîtier. Le procédé peut comprendre une étape de chauffage à une température modérée comprise entre 70°C et 90°C pendant laquelle est réalisée l'étape principale de recouvrement, afin favoriser le pliage du film et éviter les problèmes ultérieurs de retreint.

L'absence de thermoformage de nature à provoquer une déformation incontrôlée de l'impression (motif, texte) lors de l'étirement du film plastique permet d'intégrer une couche d'impression dans le film et donc de personnaliser la cassette au plus tard tout en permettant d'assurer sa protection comme nous le verrons par la suite.

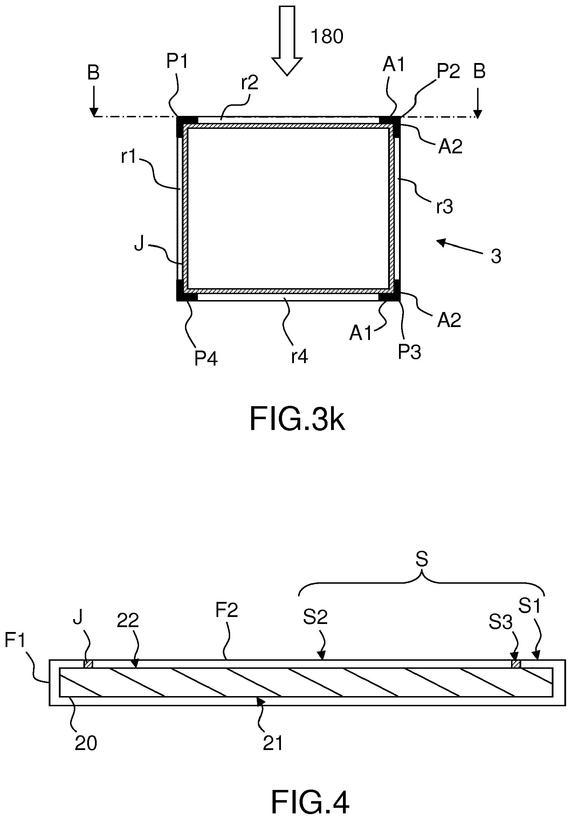

La longueur LF1 hors tout du premier film F1 est supérieure à la longueur LB de la première face 21 et la largeur IF1 hors tout du premier film est supérieure à la largeur IB de la première face 21. Par ailleurs, le film comprend sur sa périphérie, quatre parties destinées à former les quatre rabats R1 à R4. Ces quatre parties sont séparées par des entailles E1, E2, E3, E4 référencées sur la figure 4 représentant le premier film F1 seul et permettant de plier les rabats R1 à R4 indépendamment les uns des autres. Les quatre rabats R1 à R4 entourent une partie centrale CENT, délimitée par des traits pointillés sur la figure 3b, recouvrant la première face 21, par rapport à laquelle les rabats R1 à R4 peuvent être repliés indépendamment les uns des autres.

Ainsi, le premier film F1 présente, sur l'exemple non limitatif de la figure 3b, essentiellement une forme de rectangle dépourvu de ses sommets. Le premier film comprend des entailles E1 à E4 retirant les sommets du rectangle sensiblement formé par le premier film.

Sur la réalisation des figures, les entailles E1 à E4 sont sensiblement rectangulaires. Cette forme n'est pas limitative, on peut par exemple envisager des encoches en forme de quarts de cercles ou sensiblement linéaires.

Sur la réalisation non limitative des figures, la partie centrale CENT du premier film F1 est destinée à recouvrir sensiblement toute la première face sans recouvrir ses sommets qui se trouvent chacun à sensiblement à l'intersection de deux côtés adjacents CO1, CO2, CO3, CO4. En variante, la partie centrale CENT du premier film F1 est destinée à recouvrir toute la première face 21.

L'étape 100 de recouvrement de la première grande face 21 par le premier film F1 est suivie d'une étape de 110 de recouvrement des quatre petites faces 23 à 26 par le premier film F1 dont les rabats sont sensiblement parallèles aux petites faces 23 à 26 perpendiculaires au plan de la figure 3d. Plus précisément, l'étape 100 de recouvrement de la première grande face 21 par le premier film F1 est suivie d'une étape de pliage de chacun des rabats R1 à R4 de sorte qu'il vienne recouvrir une des quatre petites faces 23 à 26. Chaque rabat 23 recouvre avantageusement une seule des quatre petites faces du boîtier de la cassette 3.

Le pliage de chaque rabat R1 à R4 est réalisé le long d'un côté CO1 à CO4 de la première grande face 21 de sorte que le premier film épouse la forme de ce côté.

Avantageusement, chaque rabat R1 à R4 déborde de la petite face 23 à 26 qu'il recouvre, de sorte qu'il puisse venir recouvrir la deuxième plus grande face 22. Autrement dit, la largeur de chaque rabat R1 à R4 est supérieure à l'épaisseur e du boîtier 20 prise selon la direction perpendiculaire au deux plus grandes faces.

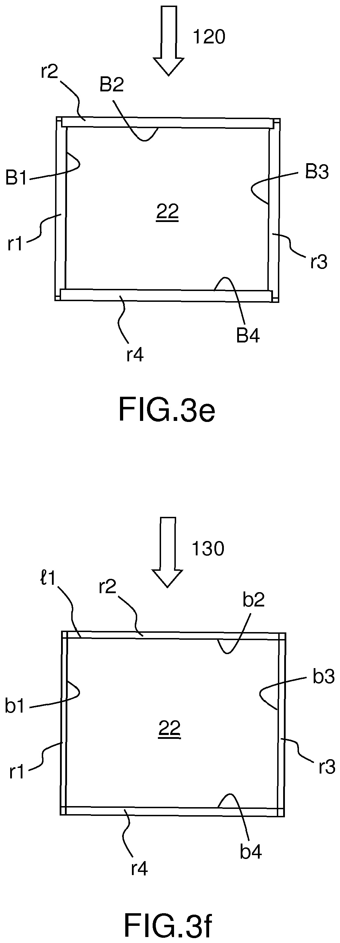

Avantageusement, l'étape 110 de recouvrement des quatre petites faces 23 à 26 par le premier film F1 est suivie d'une étape 120 de pliage d'un reste r1 à r4, de chaque rabat R1 à R4, débordant de la petite face 23 à 26 recouverte par le rabat R1 à R4 de sorte que le reste r1 à r4 du rabat R1 à R4 vienne déborder sur la deuxième grande face 22 comme visible sur la figure 3e. Il est à noter qu'il serait difficile de faire déborder le premier film recouvrant la première plus grande face 21 sur la deuxième plus grande face 22 par thermoformage sans provoquer un amincissement du film pouvant aller jusqu'à le trouer.

Dans l'exemple non limitatif des figures, le procédé comprend une étape 130, de découpage et de retrait de bords B1, B2, B3, B4 des restes des rabats r1, r2, r3, r4 recouvrant la deuxième grande face 22, selon une ligne de découpe l1 fermée, représentée sur la figure 3f, de position prédéterminée par rapport à la deuxième grande face 22. Cette ligne de découpage l1 est avantageusement sensiblement rectangulaire, comme représenté, sur la figure 3f. Cette étape réduit la taille des restes des rabats r1, r2, r3, r4 et laisse libres des nouveaux bords b1, b2, b3, b4 des restes des rabats.

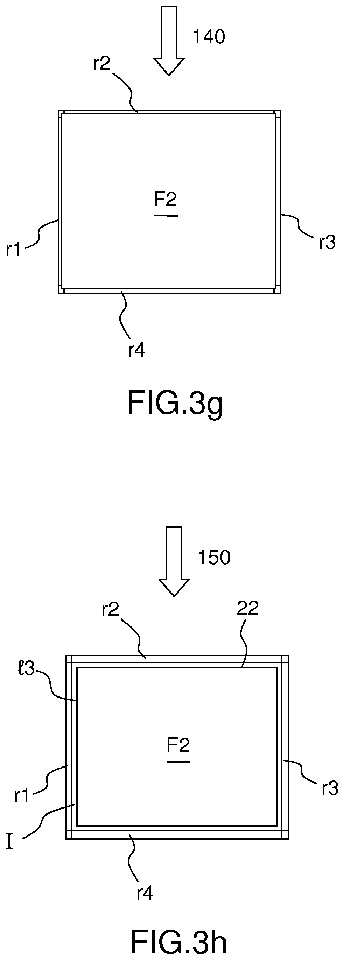

L'étape 130 est suivie d'une étape 140 de recouvrement de la deuxième grande face 22 du deuxième film F2, visible sur la figure 3g, de sorte que le deuxième film F2 recouvre une partie centrale de la deuxième grande face 22 et déborde sur les restes des rabats de façon à recouvrir totalement les nouveaux bords b1, b2, b3, b4 des restes des rabats r1, r2, r3, r4.

Avantageusement, chacune des petites faces est recouverte d'un seul film pris parmi les films F1 et F2.

Avantageusement, le deuxième film F2 recouvre uniquement la deuxième face 22 de la cassette 3 et la jonction entre les films F1 et F2 se trouve sur la deuxième grande face 22 ce qui facilite la réalisation industrielle du procédé par rapport à une jonction des films sur les flancs, c'est-à-dire sur les petites faces 23 à 26 du boîtier, et ce qui favorise la robustesse du revêtement aux agressions extérieures, le boîtier étant moins sollicité sur ses grandes faces.

En variante, les deux films F1 et F2 se recouvrent sur les petites faces ou flancs du boîtier 20.

L'étape 140 est suivie d'une étape 150 de découpe du bord du deuxième film F2 selon une deuxième ligne de découpe l2 fermée, visible sur la figure 3h, et de retrait de ce bord de position et de forme prédéterminée par rapport à la première ligne de découpe l1 de sorte que les deux films ne se recouvrent pas. L'absence de recouvrement des films permet de limiter les risques d'arrachement de l'emballage. Ce procédé permet de maitriser l'espacement entre les deux films. Un interstice I présentant une forme de boucle fermée entourant complètement le deuxième film F2.

Avantageusement, la ligne de découpage l2 présente sensiblement la même forme, sensiblement dans les mêmes proportions que la ligne l1 ainsi qu'un même centre. La ligne l2 délimite une zone plus petite que la ligne L1 afin d'éviter tout recouvrement entre les films F1 et F2. Ainsi, l'espacement entre les deux films F1 et F2 est sensiblement fixe sur tout le périmètre de la ligne de découpe l2.

Avantageusement, la taille des lignes de découpage l1 et l2 est telle que l'espacement entre les lignes l1 et l2 dans le plan de la face 22 est sensiblement un jeu permettant de garantir une absence de recouvrement entre les deux films. Autrement dit, les films sont découpés bord à bord.

Les étapes de découpe sont avantageusement des étapes de découpe laser, le laser étant déplacé par un outillage par rapport à un support selon une même ligne fermée fixe par rapport au support, lors des deux étapes de découpe, le boîtier occupant une même position et une même orientation par rapport au support lors de ces deux étapes. Ainsi, la distance entre les lignes l1 et l2 est sensiblement égale au diamètre du spot laser sur la face du boîtier. Le diamètre du spot est de l'ordre de 100 micromètres. Ce procédé présente de très hautes précisions de découpe et d'alignement de l'ordre de 10 micromètres.

En variante, les étapes de découpe sont des étapes de découpe mécanique.

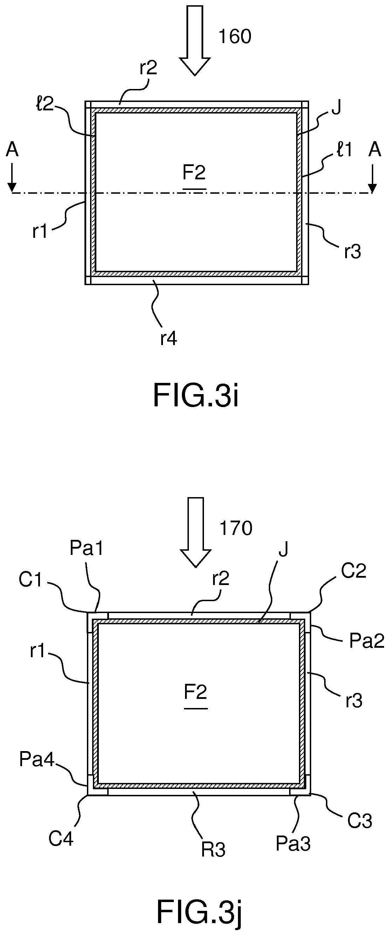

Avantageusement, le procédé comprend une étape 160 de colmatage consistant à appliquer un joint J, visible sur la figure 3i, pour combler l'interstice I formé entre le premier film F1 et le deuxième film F2, lors de l'étape 140. Cette étape permet d'assurer l'étanchéité du revêtement R tout en limitant la présence de zones d'accumulation de poussières ou de liquides et donc de germes ou de bactéries ce qui facilite le nettoyage de la cassette. Cette configuration garantit une bonne résistance mécanique de l'enveloppe car elle évite le recouvrement des films et par conséquent la présence d'un bord d'un des films sur l'autre film favorisant l'arrachement du film en surépaisseur.

Le joint J est déposé sur tout le périmètre de l'interstice I formé entre les deux films F1 et F2.

Sur la réalisation des figures, le joint J comble totalement l'interstice I. Le joint J affleure les surfaces libres du premier film F1 et du deuxième film F2. Une surface S sensiblement plane continue est formée par la surface libre S3 du joint et par les surfaces libres S1 et S2 des films F1 et F2 au voisinage du joint comme représenté sur la figure 4 représentant une coupe de la cassette selon un plan de coupe A-A. Cela permet encore de limiter la présence de zones d'accumulation de poussières ou de liquides et donc de germes ou de bactéries et d'améliorer la résistance mécanique de l'enveloppe.

En variante, le joint J comble partiellement l'interstice I.

Les films F1 et F2 présentent avantageusement sensiblement la même épaisseur.

En variante, le procédé comprend une étape de fusion des bords des deux films F1 et F2 de façon à assurer une continuité de matière entre les deux films et ainsi limiter les zones d'accumulation de poussière et de bactéries.

Avantageusement, dans ce cas, les deux films se recouvrent avant l'étape de fusion. Par exemple, le procédé ne comprend pas l'étape de découpe 130 et/ou l'étape de découpe 150. En variante, le procédé comprend les étapes de découpe 130 et 150.

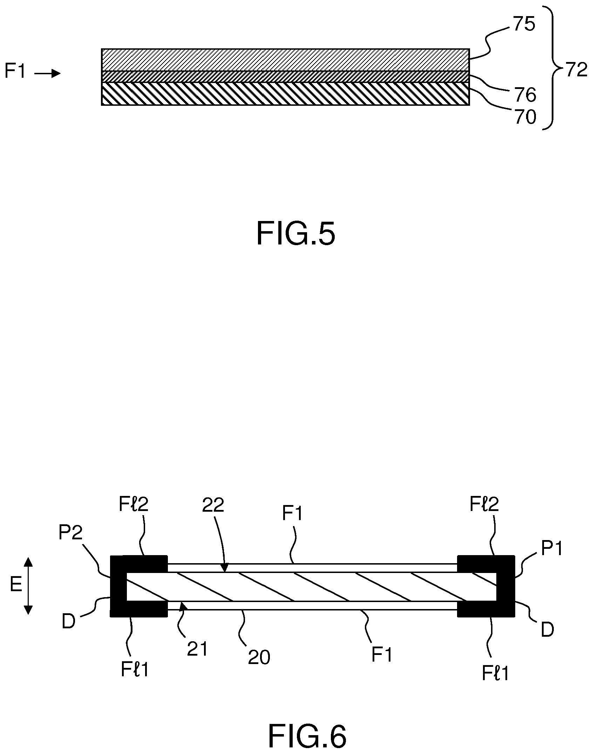

Sur la figure 5, on a représenté un exemple de réalisation du premier film F1. La structure du deuxième film F2 n'est pas représentée mais est avantageusement similaire à celle du film F1.

Le film F1 est multicouche et comprend une couche de film plastique 70 et une couche de matériau d'assemblage 75 permettant de faire adhérer le film plastique 70 au boîtier 20. Autrement dit, le film F1 et le boîtier 20 sont assemblés par adhésion directe. Le film F1 est alors accolé au boîtier 20. Les films sont positionnés, lors de étapes de recouvrement de sorte que la couche de matériau d'assemblage ou matériau adhésif 75 est interposée entre le film plastique 70 et la face recouverte par le film de façon à faire adhérer le film F1 et le boîtier 20.

Le film plastique présente une épaisseur comprise entre 150 µm et 700 µm ; par exemple 200 µm et la couche de matériau d'assemblage présente une épaisseur comprise entre 50 µm et 300 µm, par exemple 130 µm.

Avantageusement, la couche de matériau d'assemblage s'étend sur toute la surface du film.

En variante, le film F1 comprend une couche de ruban adhésif double face comprenant un ruban enduit d'une couche de matériau adhésif sur chacune de ses deux faces opposées dont une face est fixée au film plastique 70 et dont une deuxième face est destinée à faire face au boîtier 20. Avantageusement, le ruban est opaque au spectre visible et transparent aux rayons X. Ainsi, la couleur extérieure du boîtier est celle du ruban si l'adhésif est transparent.

Avantageusement, le matériau d'assemblage 75 est un solide mou qui fait adhérer le film plastique 70 au boîtier 20 par déformation de l'adhésif sous l'effet d'une pression. Cette solution présente l'avantage de ne pas nécessiter obligatoirement de chauffage et d'être rapide.

Le procédé peut comprendre une étape de chauffage à une température comprise dans un intervalle de température dans lequel le film plastique est apte à être déformé de manière stable. Cette température est par exemple comprise entre 70°C et 90°C. Cela permet d'obtenir un placage du film plastique 70 sans défauts (bulles d'air) et sans risque de décollement dans le temps.

L'étape de chauffage est, par exemple, mise en œuvre pendant l'étape 110 pour favoriser le conformage du film et le placage du film sur les flancs.

En variante, le matériau d'assemblage 75 est une colle, par exemple une colle à résine thermodurcissable. Elle fait adhérer le film et le boîtier par durcissement, par exemple par réticulation, de la colle.

Alternativement, le procédé comprend une étape d'application d'un matériau adhésif, par exemple une colle, sur les faces du boîtier 20 précédent les étapes de recouvrement du boîtier par les films. Cette configuration rend plus difficile la maitrise de l'épaisseur de la cassette sur toute sa surface. Or, on cherche à obtenir une cassette présentant une surface la plus lisse possible et une épaisseur sensiblement constante sur toute sa surface.

Avantageusement, au moins un film comprend au moins une couche d'impression 76 disposée entre le film plastique 70 et la couche de matériau d'assemblage 75 ce qui permet d'assurer une protection de la couche d'impression par le film plastique et ainsi d'améliorer la robustesse de l'impression. Cette couche d'impression est une couche d'encre, déposée par exemple par sérigraphie. L'impression est destinée à personnaliser le boîtier. Elle peut comprendre au moins un texte et/ou au moins un motif comme, par exemple, une croix dans l'exemple des figures. Avantageusement, le film plastique 70 est transparent à au moins une partie du spectre visible de façon que l'impression soit visible lorsque le film plastique est collé sur le boîtier. Cette configuration permet d'envisager de multiples possibilités de personnalisation du boîtier avec des motifs et couleurs différentes sur les différentes faces du boîtier sans opération de personnalisation face par face après emballage du boîtier.

L'épaisseur totale de la couche d'impression est comprise entre 10 µm et 50 µm. Elle fait par exemple une épaisseur de 10 µm.

Le film F2 peut être interrompu au niveau de l'embrèvement 30, notamment au niveau du connecteur 32 afin de ne pas recouvrir le connecteur 32. De plus, il n'est pas nécessaire de recouvrir le fond de l'embrèvement 30, fond parallèle à la face 21. En effet, ce fond n'est pas soumis aux mêmes agressions mécaniques que les faces externes 21 et 22 car protégé par la batterie 9. Le fond de l'embrèvement 30 peut être laissé brut ou recouvert d'une peinture.

De façon générale au moins un film peut comprendre, avant l'étape de recouvrement d'une face du boîtier par le film, au moins une ouverture, par exemple délimitée par une courbe fermée destinée à venir en regard de zones du boîtier destinées à être laissées libres par le procédé d'emballage, c'est-à-dire par le revêtement. Cette caractéristique permet d'éviter une découpe du film ultérieure au recouvrement du boîtier pour libérer ces zones. La présence de ces ouvertures préalables n'est pas permise lors d'une mise sous vide. En effet, la mise sous vide nécessite une étanchéité parfaite. Dans le cas du thermoformage, la feuille de film plastique participe à l'étanchéité donc si elle est perforée (trou ou ouverture) le vide ne peut pas être atteint. Il n'est donc pas possible de faire de découpes préalables du film.

Les films F1 et F2 masquent avantageusement des moyens de fixation du type vis ou écrou V. Ce masquage permet d'éviter toute accumulation d'impuretés dans les moyens de fixation lors de l'utilisation de la cassette 3. Le nettoyage de la cassette 3 s'en trouve facilité. Le masquage permet également de limiter l'ouverture de la cassette 3 par une personne non autorisée.

Le procédé comprend éventuellement, comme visible sur la figure 3j, une étape 170 de découpage et retrait de bords du premier film entourant les coins du boîtier de façon à laisser libres des parties Pa1 à Pa4 du boîtier comprenant et entourant les coins C1, C2, C3, C4.

Le procédé comprend ensuite, comme visible sur la figure 3k, une étape de recouvrement 180 des coins C1, C2, C3, C4 et plus précisément des parties Pa1 à Pa4 du boîtier 20 par des protections de coins P1, P2, P3, P4 de sorte à obtenir la cassette radiologique 3. Les protections de coins permettent de protéger mécaniquement les coins. Elles ont une fonction de renfort.

Le boîtier 20 de la cassette 3 est recouvert d'un revêtement R comprenant les films F1 et F2, les protections de coins P1 à P4 et le joint J.

Avantageusement, les protections de coins recouvrent les parties libres Pa1 à Pa4 sans recouvrir les films plastiques. Cela permet de limiter l'épaisseur totale de la cassette qui est normalisée et de limiter les risques d'accroche des protections de coins et limitant ainsi les risques de blesser des patients.

Le procédé peut comprendre une étape de dépôt d'un joint pour combler un espace entre au moins un film plastique et une protection de coin.

Chaque protection de coin P1, P2, P3, P4 est configurée et disposée en regard d'un coin C1, C2, C3 ou C4 du boîtier 20 de façon à recouvrir le coin correspondant C1, C2, C3 ou C4. Chaque protection de coin recouvre également partiellement les faces F1 et F2.

Sur l'exemple représenté sur les figures 3k et 6, la protection de coin comprend deux ailes A1, A2 reliées entre elles de sorte à définir un angle de 90°. Les deux ailes comprennent chacune une section en U. Autrement dit, elles présentent chacune deux flancs Fl1, Fl2 reliés par un dos D. Chaque aile A1, A2 comprend un flanc Fl1 recouvrant une partie de la première grande face 21 située à proximité d'un coin et un autre flanc Fl2 recouvrant une partie de la deuxième grande face 22 située à proximité du même coin, les dos D recouvrant les parties des petites faces reliées par le coin. Chaque dos D couvre uniquement une des petites faces. Les deux ailes de chaque protection de coin se rejoignent en regard du coin qu'elles recouvrent.

Chaque protection de coin comprend, par exemple, une couche métallique. Cela confère une protection mécanique plus solide aux coins du boîtier qui sont les parties les plus sollicitées et cela facilite la réalisation de la protection de coin qui présente une forme complexe et une faible épaisseur. En variante, les protections de coins peuvent par exemple une couche de matière plastique. Ils peuvent par exemple présenter la même structure et composition que les films F1 et F2. La protection de coin comprend avantageusement une couche de matériau d'assemblage.

Avantageusement, chaque protection de coins P1, P2, P3, P4 présente une épaisseur définie de façon que lorsque la plaque est posée sur un support plan, elle repose sur le support plan uniquement par les protections de coins. Cela permet de limiter l'exposition des films aux agressions mécaniques (frottements) et/ou chimiques (produits) en évitant le contact des films avec le support. L'épaisseur des protections de coins est comprise entre 300 µm et 400 µm. Cette épaisseur est l'épaisseur des flancs Fl1 et Fl2.

Les films F1 et F2 présentent typiquement une épaisseur comprise entre 200 µm et 1000 µm. Elle est par exemple de 330 µm.

Lors de l'étape de recouvrement des coins par les protections de coins, les protections de coins sont fixées au boîtier. Les protections de coins peuvent être multicouches et comprendre une couche d'assemblage comme décrit précédemment ou bien le procédé peut comprendre une étape d'application d'une couche d'assemblage sur le coin préalablement à l'étape de recouvrement d'un coin par le boîtier.

La face 21 est destinée à être traversée par le rayonnement ionisant à détecter. Elle est transparente au rayonnement de façon à permettre au détecteur de recevoir le rayonnement. Le film F1 recouvrant cette face ou au moins sa partie centrale, est également transparent au rayonnement ionisant à détecter.

Le boîtier 20 peut comprendre une enveloppe réalisée dans une pièce mécanique monobloc formant cinq premières faces de la forme essentiellement parallélépipédique dont les deux plus grandes faces, et un bouchon pour former une sixième face de la forme essentiellement parallélépipédique. Dans ce cas, le premier film peut comprendre seulement 3 rabats susceptibles d'être replié indépendamment des autres rabats pour venir recouvrir une des faces latérales du boîtier 20. De façon plus générale, pour éviter le thermoformage, le procédé comprend une étape de recouvrement d'une grande face du boîtier par un premier film de sorte à comprendre au moins deux rabats susceptibles d'être repliés indépendamment l'un de l'autre pour venir recouvrir deux faces latérales adjacentes du boîtier 20.

Sur la réalisation de la figure 2, la première face 21 est sensiblement plane et la deuxième face 22 est comprend l'embrèvement. En variante, la deuxième face 22 est sensiblement plane et la première face est comprend l'embrèvement 30.

Par le passé, la radiologie médicale utilisait des films argentiques qui étaient manipulés dans des cassettes. La norme ISO 4090 a défini les dimensions des cassettes enveloppant les films argentiques. L'épaisseur E des cassettes, définie par la norme, est comprise entre 13 et 16 mm. Avantageusement, la cassette 3 selon l'invention répond, quant à ses dimensions, aux exigences de la norme ISO 4090. Plus particulièrement, l'épaisseur hors tout de la cassette 3 est inférieure à 16 mm. Ceci permet d'utiliser les moyens de rangements de cassettes argentiques pour une cassette numérique 3 selon l'invention.

## Revendications

1. Procédé d'emballage d'un boîtier (20) présentant une forme essentiellement parallélépipédique définie par des faces principales dont une première plus grande face (21) et une deuxième plus grande face (22) et des faces latérales (23, 24, 25, 26), les faces latérales étant séparées deux à deux par des coins du boîtier, le boîtier logeant un détecteur numérique de rayonnement ionisant (5) sous forme d'un panneau plat, le procédé étant **caractérisé en ce qu'**il comprend une étape principale de recouvrement du boîtier par plusieurs films comprenant une première étape (100) de recouvrement de la première plus grande face (21) du boîtier (20) par un premier film (F1) configuré, positionné et orienté par rapport à la première plus grande face (21) de sorte à comprendre des rabats (R1, R2, R3, R4) débordant de la première plus grande face (21) et aptes à être repliés indépendamment l'un de l'autre ou les uns des autres pour venir recouvrir des faces latérales adjacentes (23, 24, 25, 26).

2. Procédé selon la revendication précédente, dans lequel le premier film (F1) comprend au moins trois rabats (R1, R2, R3) débordant de la première grande face (21) et aptes à être repliés indépendamment les uns des autres pour venir recouvrir au moins trois des faces latérales (23, 24, 25) du boîtier (20).

3. Procédé selon l'une quelconque des revendications précédentes, comprenant une étape (110) de pliage des rabats (R1, R2, R3) de sorte que les rabats viennent recouvrir des faces latérales adjacentes du boîtier.

4. Procédé selon la revendication précédente, dans lequel les rabats débordent des faces latérales adjacentes, le procédé comprenant une étape (120) de pliage des rabats de sorte qu'un reste des rabats (r1, r2, r3, r4) vienne recouvrir la deuxième plus grande face.

5. Procédé selon l'une quelconque des revendications précédentes, dans lequel l'étape principale de recouvrement comprend une étape recouvrement de la deuxième plus grande face (22) par un deuxième film (F2), le deuxième film (F2) recouvrant uniquement la deuxième plus grande face (22).

6. Procédé selon la revendication précédente, dans lequel l'étape principale de recouvrement est réalisée de sorte que les deux films ne se recouvrent pas, le procédé comprenant une étape de colmatage d'un interstice entre le premier film (F1) et le deuxième film (F2) par un joint (J).

7. Procédé selon la revendication précédente, dans lequel l'étape principale de recouvrement comprend une étape de découpage et de retrait de bords des restes des rabats (r1, r2, r3, r4) recouvrant la deuxième grande face (22) selon une ligne de découpe (l1) de position prédéterminée par rapport à la deuxième plus grande face (22) et une étape de découpe et de retrait d'un bord du deuxième film (F2) selon une deuxième ligne de découpe (l2) fermée de position et de forme prédéterminée par rapport à la première ligne de découpe (l1).

8. Procédé selon l'une quelconque des revendications précédentes, comprenant une étape de recouvrement des coins du boîtier par des protections de coins (P1, P2, P3, P4).

9. Procédé selon la revendication précédente, comprenant une étape de découpage et de retrait de bords du premier film (F1) entourant des coins du boîtier de façon à laisser libres des parties du boîtier comprenant et entourant des coins du boîtier, les protections de coins étant disposées de façon à recouvrir les parties libres sans recouvrir le premier film (F1).

10. Procédé selon l'une quelconque des revendications 8 à 9, dans lequel les épaisseurs de chaque protection de coin et de chaque film sont choisies de sorte que lorsque la cassette est posée sur un support plan, seules les protections de coins sont en contact avec le support plan.

11. Procédé selon l'une quelconque des revendications précédentes, dans lequel au moins un film comprend un film plastique, une couche d'assemblage permettant de faire adhérer le premier film au boîtier et une couche d'impression interposée entre un film plastique et la couche d'assemblage.

12. Cassette radiologique portable comprenant un boîtier (20) présentant une forme essentiellement parallélépipédique définie par des faces principales dont une première plus grande face (21) et une deuxième plus grande face (22) et des faces latérales (23, 24, 25, 26), les faces latérales étant séparées deux à deux par des coins du boîtier, le boîtier logeant un détecteur numérique de rayonnement ionisant (5) sous forme d'un panneau plat, la cassette radiologique étant **caractérisée en ce que** le boîtier est recouvert par plusieurs films comprenant un premier film recouvrant la première plus grande face (21), le premier film comprenant des rabats débordant de la première plus grande face (21) et étant repliés indépendamment l'un de l'autre ou les uns des autres pour recouvrir des faces latérales adjacentes (23, 24, 25, 26).

13. Cassette radiologique selon la revendication précédente, dans lequel un reste de chaque rabat recouvre la deuxième plus grande face.

14. Cassette radiologique selon la revendication précédente, comprenant un deuxième film recouvrant uniquement la deuxième plus grande face.

15. Cassette radiologique selon la revendication précédente, comprenant un joint colmatant un interstice entre le premier film et le deuxième film.

16. Cassette radiologique selon la revendication précédente, comprenant des protections de coins (P1, P2, P3, P4) recouvrant les coins.

17. Cassette radiologique selon la revendication précédente, dans laquelle les protections de coins ne recouvrent pas les films.

18. Cassette radiologique selon la revendication précédente, dans lequel l'épaisseur de chaque protection de coin et de chaque film est choisie de sorte que lorsque la cassette est posée sur un support plan, seules les protections de coins sont en contact avec le support plan.

19. Cassette radiologique selon l'une quelconque des revendications 12 à 18, dans lequel au moins un film comprend un film plastique, une couche d'assemblage permettant de faire adhérer le premier film au boîtier et une couche d'impression interposée entre un film plastique et la couche d'assemblage.

## Patentansprüche

1. Verfahren zum Verpacken eines Gehäuses (20) mit einer im Wesentlichen quaderförmigen Gestalt, definiert durch Hauptflächen, darunter eine erste größte Fläche (21) und eine zweite größte Fläche (22) und Seitenflächen (23, 24, 25, 26), wobei die Seitenflächen paarweise durch Ecken des Gehäuses getrennt werden, wobei das Gehäuse einen digitalen Ionisierungsstrahlendetektor (5) in Form einer flachen Platte aufnimmt, wobei das Verfahren **dadurch gekennzeichnet ist, dass** es eine Hauptfläche des Bedeckens des Gehäuses mit einer Mehrzahl von Folien umfasst, umfassend einen ersten Schritt (100) des Bedeckens der ersten größten Fläche (21) des Gehäuses (20) mit einem ersten Film (F1), so mit Bezug auf die erste größte Fläche (21) konfiguriert, positioniert und orientiert, dass er Laschen (R1, R2, R3, R4) umfasst, die die erste größte Fläche (21) überschreiten und unabhängig voneinander gefaltet werden können, um benachbarte Seitenflächen (23, 24, 25, 26) zu bedecken.

2. Verfahren nach dem vorherigen Anspruch, bei dem der erste Film (F1) wenigstens drei Laschen (R1, R2, R3) umfasst, die die erste größte Fläche (21) überschreiten und unabhängig voneinander gefaltet werden können, um wenigstens drei Seitenflächen (23, 24, 25) des Gehäuses (20) zu bedecken.

3. Verfahren nach einem der vorherigen Ansprüche, das einen Schritt (110) des Faltens der Laschen (R1, R2, R3) beinhaltet, so dass die Laschen benachbarte Seitenflächen des Gehäuses bedecken.

4. Verfahren nach dem vorherigen Anspruch, bei dem die Laschen benachbarte Seitenflächen überschreiten, wobei das Verfahren einen Schritt (120) des Faltens der Laschen beinhaltet, so dass ein Rest der Laschen (r1, r2, r3, r4) die zweite größte Fläche bedeckt.

5. Verfahren nach einem der vorherigen Ansprüche, bei dem der Hauptschritt des Bedeckens einen Schritt des Bedeckens der zweiten größten Fläche (22) mit einem zweiten Film (F2) beinhaltet, wobei der zweite Film (F2) nur die zweite größte Fläche (22) bedeckt.

6. Verfahren nach dem vorherigen Anspruch, bei dem der Hauptschritt des Bedeckens so durchgeführt wird, dass sich die beiden Filme nicht bedecken, wobei das Verfahren einen Schritt des Ausfüllens eines Zwischenraums zwischen dem ersten Film (F1) und dem zweiten Film (F2) mit einer Dichtung (J) beinhaltet.

7. Verfahren nach dem vorherigen Anspruch, bei dem der Hauptschritt des Bedeckens einen Schritt des Abschneidens und Entfernens von Rändern der Reste der Laschen (r1, r2, r3, r4), die die zweite größte Fläche (22) bedecken, entlang einer Schnittlinie (I1) von einer vorbestimmten Position mit Bezug auf die zweite größte Fläche (22) und einen Schritt des Abschneidens und Entfernens eines Rands des zweiten Films (F2) entlang einer zweiten geschlossenen Schnittlinie (12) mit einer vorbestimmten Position und Form mit Bezug auf die erste Schnittlinie (I1) beinhaltet.

8. Verfahren nach einem der vorherigen Ansprüche, das einen Schritt des Bedeckens der Ecken des Gehäuses mit Eckenschutzvorrichtungen (P1, P2, P3, P4) beinhaltet.

9. Verfahren nach dem vorherigen Anspruch, das einen Schritt des Abschneidens und Entfernens von Rändern des ersten Films (F1) beinhaltet, die Ecken des Gehäuses so umgeben, dass sie Teile des Gehäuses freilassen, die Ecken des Gehäuses umfassen und umgeben, wobei die Gehäuseschutzvorrichtungen so angeordnet sind, dass sie die freien Teile bedecken, ohne den ersten Film (F1) zu bedecken.

10. Verfahren nach einem der Ansprüche 8 bis 9, bei dem die Dicken jeder Eckenschutzvorrichtung und jedes Films so gewählt werden, dass, wenn die Kassette auf einen ebenen Träger gelegt wird, nur die Eckenschutzvorrichtungen mit dem ebenen Träger in Kontakt sind.

11. Verfahren nach einem der vorherigen Ansprüche, bei dem wenigstens ein Film einen Plastikfilm, wobei eine Montageschicht es zulässt, den ersten Film auf das Gehäuse zu kleben, und eine Druckschicht zwischen einem Plastikfilm und der Montageschicht umfasst.

12. Tragbare Röntgenkassette, umfassend ein Gehäuse (20) mit einer im Wesentlichen quaderförmigen Gestalt, definiert durch Hauptflächen, darunter eine erste größte Fläche (21) und eine zweite größte Fläche (22) und Seitenflächen (23, 24, 25, 26), wobei die Seitenflächen paarweise durch Ecken des Gehäuses getrennt sind, wobei das Gehäuse einen digitalen Ionisierungsstrahlendetektor (5) in Form einer flachen Platte aufnimmt, wobei die Röntgenkassette **dadurch gekennzeichnet ist, dass** das Gehäuse von einer Mehrzahl von Filmen bedeckt wird, umfassend einen ersten Film, der die erste größere Fläche (21) bedeckt, wobei der erste Film Laschen umfasst, die die erste größere Fläche (21) überschreiten und unabhängig voneinander gefaltet sind, um benachbarte Seitenflächen (23, 24, 25, 26) zu bedecken.

13. Röntgenkassette nach dem vorherigen Anspruch, bei der ein Rest jeder Lasche die zweite größte Fläche bedeckt.

14. Röntgenkassette nach dem vorherigen Anspruch, die einen zweiten Film umfasst, der nur die zweite größte Fläche bedeckt.

15. Röntgenkassette nach dem vorherigen Anspruch, die eine Dichtung aufweist, die einen Zwischenraum zwischen dem ersten Film und dem zweiten Film ausfüllt.

16. Röntgenkassette nach dem vorherigen Anspruch, die Eckenschutzvorrichtungen (P1, P2, P3, P4) umfasst, die die Ecken bedecken.

17. Röntgenkassette nach dem vorherigen Anspruch, wobei die Eckenschutzvorrichtungen die Filme nicht bedecken.

18. Röntgenkassette nach dem vorherigen Anspruch, bei der die Dicke jeder Eckenschutzvorrichtung und jedes Films so gewählt wird, dass, wenn die Kassette auf einen flachen Träger gelegt wird, nur die Eckenschutzvorrichtungen mit dem ebenen Träger in Kontakt sind.

19. Röntgenkassette nach einem der Ansprüche 12 bis 18, bei der wenigstens ein Film einen Plastikfilm, eine Montageschicht, die es zulässt, den ersten Film auf das Gehäuse zu kleben, und eine Druckschicht zwischen einem Plastikfilm und der Montageschicht umfasst.

## Claims

1. Method for packaging a housing (20) having an essentially parallelepipedal form defined by main faces including a first larger face (21) and a second larger face (22) and lateral faces (23, 24, 25, 26), the lateral faces being separated in pairs by corners of the housing, the housing housing a digital detector of ionizing radiation (5) in the form of a flat panel, the method being **characterized in that** it comprises a main step of covering of the housing by a plurality films comprising a first step (100) of covering of the first larger face (21) of the housing (20) by a first film (F1) configured, positioned and oriented relative to the first larger face (21) so as to include flaps (R1, R2, R3, R4) extending beyond the first larger face (21) and capable of being folded independently of one another to cover adjacent lateral faces (23, 24, 25, 26).

2. Method according to the preceding claim, in which the first film (F1) includes at least three flaps (R1, R2, R3) extending beyond the first large face (21) and capable of being folded independently of one another to cover at least three of the lateral faces (23, 24, 25) of the housing (20).

3. Method according to any one of the preceding claims, comprising a step (110) of folding of the flaps (R1, R2, R3) such that the flaps cover adjacent lateral faces of the housing.

4. Method according to the preceding claim, in which the flaps extend beyond adjacent lateral faces, the method comprising a step (120) of folding of the flaps such that a remainder of the flaps (r1, r2, r3, r4) covers the second larger face.

5. Method according to any one of the preceding claims, in which the main covering step comprises a step of covering of the second larger face (22) by a second film (F2), the second film (F2) covering only the second larger face (22).

6. Method according to the preceding claim, in which the main covering step is performed such that the two films do not overlap, the method comprising a step of sealing of an interstice between the first film (F1) and the second film (F2) by a seal (J).

7. Method according to the preceding claim, in which the main covering step comprises a step of cutting and of removal of the edges of the remainders of the flaps (r1, r2, r3, r4) covering the second larger face (22) along a cutting line (11) of predetermined position relative to the second larger face (22) and a step of cutting and of removal of an edge of the second film (F2) along a closed second cutting line (12) of predetermined position and form relative to the first cutting line (l1).

8. Method according to any one of the preceding claims, comprising a step of covering of the corners of the housing by corner protections (P1, P2, P3, P4).

9. Method according to the preceding claim, comprising a step of cutting and of removal of edges of the first film (F1) surrounding corners of the housing so as to leave free parts of the housing including and surrounding corners of the housing, the corner protections being arranged so as to cover the free parts without covering the first film (F1).

10. Method according to any one of Claims 8 to 9, in which the thicknesses of each corner protection and of each film are chosen such that, when the cassette is placed on a flat support, only the corner protections are in contact with the flat support.

11. Method according to any one of the preceding claims, in which at least one film comprises a plastic film, an assembling layer making it possible to make the first film adhere to the housing and a printing layer interposed between a plastic film and the assembly layer.

12. Portable radiological cassette comprising a housing (20) having an essentially parallelepipedal form defined by main faces including a first larger face (21) and a second larger face (22) and lateral faces (23, 24, 25, 26), the lateral faces being separated in pairs by corners of the housing, the housing housing a digital detector of ionizing radiation (5) in the form of a flat panel, the radiological cassette being **characterized in that** the housing is covered by a plurality of films comprising a first film covering the first larger face (21), the first film including flaps extending beyond the first larger face (21) and being folded independently of one another to cover adjacent lateral faces (23, 24, 25, 26).

13. Radiological cassette according to the preceding claim, in which a remainder of each flap covers the second larger face.

14. Radiological cassette according to the preceding claim, comprising a second film covering only the second larger face.

15. Radiological cassette according to the preceding claim, including a seal sealing an interstice between the first film and the second film.

16. Radiological cassette according to the preceding claim, including corner protections (P1, P2, P3, P4) covering the corners.

17. Radiological cassette according to the preceding claim, in which the corner protections do not cover the films.

18. Radiological cassette according to the preceding claim, in which the thickness of each corner protection and of each film is chosen such that, when the cassette is placed on a flat support, only the corner protections are in contact with the flat support.

19. Radiological cassette according to any one of Claims 12 to 18, in which at least one film comprises a plastic film, an assembly layer making it possible to make the first film adhere to the housing and a printing layer interposed between a plastic film and the assembly layer.
